(19) Europäisches Patentamt
European Patent Office
Office européen des brevets

(11) EP 1 791 970 B1

(12) **EUROPEAN PATENT SPECIFICATION**

(45) Date of publication and mention
of the grant of the patent:
**17.11.2010 Bulletin 2010/46**

(51) Int Cl.:
**C12Q 1/04** *(2006.01)*  **G01N 21/03** *(2006.01)*

(21) Application number: **05771979.1**

(22) Date of filing: **16.08.2005**

(86) International application number:
**PCT/IL2005/000884**

(87) International publication number:
**WO 2006/018839 (23.02.2006 Gazette 2006/08)**

(54) **DETECTION OF BACTERIA IN FLUIDS**

NACHWEIS VON BAKTERIEN IN FLÜSSIGKEITEN

DETECTION DE BACTERIES DANS DES FLUIDES

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.08.2004 US 601644 P**

(43) Date of publication of application:
**06.06.2007 Bulletin 2007/23**

(73) Proprietor: **Bacterioscan**
**23100 Migdal Ha'Emek (IL)**

(72) Inventors:
• **WEICHSELBAUM, Amnon**
**32207 Haifa (IL)**
• **DE LA ZERDA, Jaime**
**34751 Haifa (IL)**
• **REGEV, Issachar**
**Galilee (IL)**

(74) Representative: **Messulam, Alec Moses**
**A. Messulam & Co. Ltd.**
**43-45 High Road**
**Bushey Heath**
**Hertfordshire WD23 1EE (GB)**

(56) References cited:
**EP-A- 1 136 563      US-A- 3 627 424**
**US-A- 5 139 031      US-A- 5 187 368**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates in general to assaying a body fluid. In particular the present invention relates to optically testing urine, for the presence of bacteria, light scattering measurements and filtration.

**BACKGROUND OF THE INVENTION**

Aqueous fluids such as solutions, emulsions or suspensions are very common in biological context. Such are potable water for human or animal consumption, liquid food or drinks, urine, amniotic or spinal fluids. Such fluids may be occasionally tested for the presence of bacteria. In clinical microbiology laboratories, a large proportion of analyzed samples are urine samples. Common analysis of urine samples involve microscopy and or culturing, require skilled operators and are time and resource consuming. Therefore, any cheap and quick screening method which could obviate a significant amount of expensive and time consuming analytical methods, would be beneficial.

Test strips for screening for urinary tract infections are commercially available. Such strips include specific reagents embedded in two distinct pads. One pad contains reagents testing for the presence of leukocyte esterase in the sample. The other pad contains reagents to analyze for nitrite thereby to test the presence of nitrite-forming bacteria. The detection of bacterial infection is accomplished by matching the colored pads with a gradation of colors in calibrated color charts. European patent application 0320154A1 discloses a method for screening of urine based on a similar approach. The method includes carrying out at least two separate assays on portions of a sample of urine mixed with specific reagents. One assay detects the presence to leucocytes and the other detects the presence of compounds generated by the bacteria such as nitrite. However both above mentioned methods fail in detecting bacteria that do not generate those products matching the specific reagents included. The methods are based on a relatively high bacterial concentration in the sample under test and therefore such screening processes are prone to insufficient sensitivity and relatively low specificity. Furthermore these screening methods are limited to specific populations of patients, they should not be applied for example in infants and or to pregnant women.

EP 1136563 discloses a method of detecting and quantitating bacteria in urine which comprises measuring fluorescence emission and forward scattering in a flow cyclometer in the presence of cationic surfactant to reduce interference by contaminants such as mucus threads, crystals, amorphous salts and cell fragments.

According to a first aspect of the present invention, there is provided a method for detecting bacteria in a fluid comprising the steps of:

   a. filtering a sample of said fluid by means of a first filter thereby excluding particles larger than said bacteria;
   b. illuminating said sample of fluid using a collimated light beam;
   c. measuring the intensity of light scattered by said sample of fluid at least at one point, and
   d. comparing said scattered light to a calibration scale.

**BRIEF DESCRIPTION OF THE DRAWINGS**

   Fig. 1 is a schematic presentation of a system for detecting bacteria in fluids according to the present invention;
   Fig. 2 is a cross sectional view in a schematic cuvette according to a preferred embodiment of the present invention;
   Fig. 3 is a cross sectional view in a schematic optical unit of a system for detecting bacteria in fluids according to a preferred embodiment of the present invention;
   Fig. 4A is an isometric view of a cuvette unit according to a preferred embodiment of the present invention;
   Fig. 4B is an exploded view of the cuvette unit shown in Fig. 4A;
   Fig. 4C is a cross sectional view in a cuvette unit according to another preferred embodiment of the present invention;
   Fig. 4D is a cross sectional view of the cuvette unit shown in Fig. 4C;
   Fig. 5 is a polar plot of a simulated angular distribution of the intensity of light scattered by two particles of different sizes;
   Fig. 6A is a graph comparing a simulated versus measured scattering profiles of a typical urine supplemented with bacteria;
   Fig. 6B is a graph comparing simulated versus measured contributions of the bacteria and the salt particles to the scattering profiles of Fig. 6A;
   Fig. 7A is an angular scattering distribution measured for typical urine sample containing bacteria measuring $10^3$ CFU/ml;

Fig. 7B is an angular scattering distribution measured for typical urine sample containing $10^4$ CFU/ml;

Fig. 7C is an angular scattering distribution measured for typical urine sample containing $10^5$ CFU/ml;

Fig. 7D is an angular scattering distribution measured for typical urine sample containing $10^6$ CFU/ml;

Fig. 8 is a graph comparing measured versus fitted scattering profiles computed for the urine samples as shown in Figs 7A - 7D respectively;

Fig. 9 is a graph comparing measured versus fitted scattering profiles of an exemplary urine sample containing bacteria

## DETAILED DESCRIPTION OF THE PRESENT INVENTION

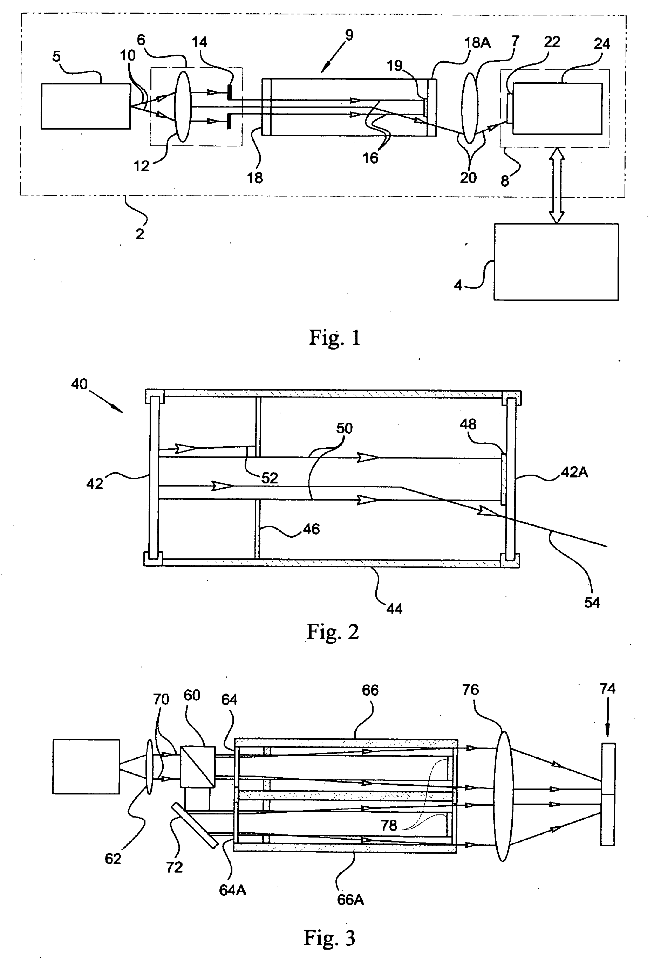

The system of the present invention provides for detecting bacteria in aqueous fluids such as water, aqueous solutions, gels, emulsions and or suspensions, liquid food and drinks, urine, spinal fluids, amniotic fluid and serum. For describing the system disclosed by the invention, reference is first made to **Fig. 1,** which shows a scheme of a system for detecting bacteria according to the present invention. The system consists of an optical unit **2** in which samples of the examined fluid are optically tested. A processor **4** linked to the optical unit carries out measurements and calculations and activates the optical unit **2.** A user interface unit, not shown, linked to processor **4,** typically consists of a display for presenting results of measurements and instructions to the operator and a keyboard for entering data. A power supply, not shown, powers the optical and operator interface units and the processor.

## Optical unit

The optical unit **2** contains a light source **5** producing a light beam. The light beam is collimated by collimator **6.** Converging simple or compound lens **7** focuses light coming from a sample of the examined fluid sample into a receiver unit **8.** A mountable cuvette unit (CU) **9** containing the sample of fluid is placed between the collimator **6** and the converging lens **7.** Several alternative structural features of the CU are described below. In general the term cuvette hereinafter means a transparent vessel capable of containing a sample of the fluid and is mountable in the optical unit.

Light rays **10** represent the illuminating beam emitted by light source **5.** The beam is collimated by a collimator **6** consisting of a simple or compound lens **12** and a diaphragm containing an aperture **14.** A beam of collimated light represented by the rays **16** propagates through window **18** and the fluid within the cuvette. Light obscuring means **19** prevents any direct illuminating light to get into receiver unit **8.** Light scattered in the fluid is represented by light ray **20** coming out of cuvette **9** through the unobscured segment of window **18A.** Scattered light is further focused by means of converging simple or compound lens **7** on the plane of detector **22** mounted in receiver unit **8.** The scattered light impinging on lens 7 is limited according to the present invention by means of the sidewalls of CU **9** and light obscuring means **19** into a predefined range of forward scattering angles. Receiver unit **8** contains electronic circuitry **24** connected to detector **22** providing for signal amplification, sampling, digitization, intermediate data storage and timing. Data related to the measured intensity of the light received by the receiving unit is further transferred to processor **4** for post-processing to be described infra.

An appropriate light source for the system may be any of the following: incandescent, gas discharge, spark and or arc lamps; solid state devices LEDS or lasers, or any laser source operative in the range of near infrared up to soft ultra violet. Preferable are light sources capable of emitting an illuminating power exceeding a minimal requirement specified by the sensitivity of the system. Optionally an excluding filter permitting a specific band of wavelengths to pass, and or a polarizing device are disposed adjacent to collimating lens **7.** The detector of the invention is typically a monolithic single detector, an array of detectors and or a monolithic detector array, operative in the wavelengths, band or bands of wavelengths, or range of wavelengths of the light source. Preferable are arrays of detectors and or monolithic detector arrays providing for measuring the intensity of scattered light over a plane.

Reference is made to **Fig. 2** schematically showing a cuvette according to a preferred embodiment of the present invention. Cuvette **40** has transparent windows **42** and **42A** disposed at two opposing sides of the cuvette respectively. The aperture in diaphragm **46** is disposed inside the cuvette close to window **42.** Light obscuring means **48** is disposed at window **42A** coaxially with diaphragm **46.** The beam of collimated light is represented by rays **50.** Light ray **52** scattered along its track after passing through window **42,** is blocked by the diaphragm **46.**

Reference is now made to **Fig. 3** in which a schematic optical unit of a system according to a preferred embodiment of the present invention is shown. A beam splitting device **60** is disposed between the collimating lens **62** and the window **64** of cuvette **66.** Two collimated beams are emitted from the beam splitting device **60.** The first beam propagates towards cuvette **66.** The second beam which is perpendicular to the first one is reflected by reflector **72** to propagate in parallel with the first beam towards cuvette **66A.** Light from each of the cuvettes **66** and **66A** is focused on the respective parts of dual receiver unit **74.**

A system for detecting bacteria in fluids also disclosed by the present invention employs two light sources such as two laser diodes. Obviating the beam splitter and the reflector described above. Optionally the lasers diodes differ in their wavelengths. The obscuring means **78** shown in Fig. 3 are optionally omitted in this preferred embodiment of the invention, thereby enhancing the sensitivity of fluorescence measurements is promoted.

**Cuvette unit**

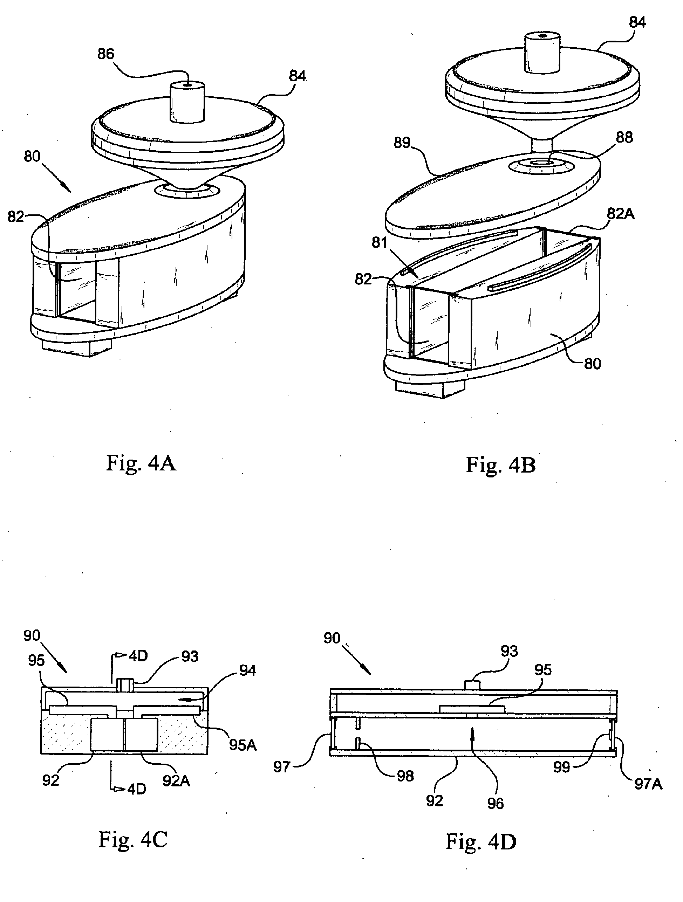

Reference is now made to **Figs 4A** and **4B** showing respectively an isometric and an exploded views of a cuvette unit (CU) according to a preferred embodiment of the invention correspondingly. The CU **80** consists of a single cuvette disposed inside the CU housing, having a window **82** disposed at a side of the CU facing the light source and a parallel window **82A** disposed at the opposite side. Optionally a filtration means **84** having an inlet aperture **86** is connected to the inlet aperture of the CU **88** located at the CU cover **89.** A cutoff filter, not shown, rejecting particles above a specified size, is located inside the filtering device **84.**

The CU and the body of the filtering device are typically made of materials, such as plastic resins, commonly used for manufacturing disposable bottles or containers. Preferably the cuvettes are made of materials compatible with the specific fluids undergoing analyses such that they do not alter the constitution of the cuvette or conversely that the cuvette does not cause changes in the examined fluid. The windows are made for example of plastic typically used for manufacturing optical lenses, glass or quartz. The refraction index homogeneity of the window, namely variations in the refraction index within the window do not exceed 0.0001. The root mean square value of the surface roughness of the windows does not exceed **1** nanometer. Preferable are windows having an optical quality of their surfaces defined by a scratch/dig number 40/20 or lower. Acceptable according to the invention are for example windows made of plastic or glass whose width does not exceed 0.5 millimeter. The optical homogeneity of the bulk of the window and or its surface roughness impacts the signal to noise ratio of a measured intensity of the scattered light and in turn the sensitivity of the system.

Reference is now made to **Figs 4C - 4D** in which two sectional views of a CU having a dual cuvette configuration according to another embodiment of the present invention are shown respectively. Such CUs are especially suited for assaying fluids such as urine. By employing two different preprocessing procedures applied to each of the samples of fluid within each cuvette, one serves as a reference sample for the other as is further described infra. CU **90** consists of a pair of cuvettes **92** and **92A** mutually attached along one of their sidewalls. CU **90** has a common inlet consisting of an aperture **93** and a space **94** located above both cuvettes for receiving the samples. Filters **95** and **95A,** optionally having each a different cutoff threshold, are disposed at the inlet of each cuvette. In **Fig. 4D** a sectional view of this CU is shown as is indicated in **Fig. 4C.** Filter **95** is disposed at inlet **96.** An aperture in diaphragm **98** is disposed close to window **97** and is coaxial with a light obscuring means **99** disposed on the inner surface of the opposing window **97A.** Cuvette **92A** is similarly furnished with windows, a filter, an aperture in diaphragm and a light obscuring means respectively.

**The Assay**

In general assaying includes three main steps as follows: (i) a preprocessing step; (ii) measurements step and (iii) a post processing step.

**(i) Preprocessing**

Urine typically contains micro particles such as salt crystals, biological macromolecules and cells, all of which have refraction indexes differing from the refraction index of the fluid. Therefore such particles may interfere with the reading of the scattering associated with the bacteria. Other biological fluids are likely to contain a multitude of interfering constituents. The preprocessing step according to the invention aims at excluding from the samples undesirable constituents, typically but not exclusively particles of sizes larger than those of the bacteria. The preprocessing step basically includes simple size exclusion, such as by mechanical filtering. Acidification, alkalization, sedimentation chemically and or by cooling and or any combination thereof, can be optionally employed in addition to the mechanical filtering. However, such additional chemical or physical treatments are aimed to exclude particles other than the bacteria and not to induce bacteria proliferation as is further described in example 4 below.

Reference is again made to **Figs 4C.** The filters **95** and **95A** are disposed at the inlet of cuvettes **92** and **92A** respectively. Their respective cutoff limits allow the passage of particles smaller than that cutoff limit. For example, in screening urine for the presence of bacteria whose size is about 5 microns, according to a preferred embodiment of the present invention, the filter **95** are such that only particles smaller than 5 microns pass through, whereas filter **95A** transmits particles sized not more than **1** micron. A sample of urine is pressurized into cuvettes **92** and **92A** through aperture **93.** Pressurizing is effected by means of an injector such as a syringe. The pressurized urine passes from the space **94** into each of both cuvettes through its corresponding filter. As a result cuvette **92** contains the urine and bacteria at a concentration level of about the original bacterial concentration level. On the other hand cuvette **92A** owing to its lower cutoff size filter, hardly contains bacteria but mainly contains other scattering particles such as crystals as are similarly present in the other cuvette.

Optionally reagents are introduced for potential interaction with the examined fluid, either before dispensing in the CU or in the CU. Such reagents consist of chemical and or biochemical reactants potentially effecting chemical or biochemical reactions either with the bacteria or with other constituents of the liquid. However such reagents and interactions only provide for diminishing the obscuring effects of particles other than the bacteria and do not include any nourishing compounds and/or any incubation processes normally inducing bacteria proliferation. Such activities normally providing for bacteria proliferation are excluded according to the present invention and are referred hereinafter as incubation. The products of such reactions are of specific optical features that enable differentiating between bacteria and the other particulate matter. Alternatively such reagents are able to chemically or bio-chemically interact with other particles such as the biological compounds and the organic cells in order to promote their aggregation and or sedimentation.

**(ii) Measurements**

Subsequently, the CU containing the analyzed fluid is mounted into, the optical unit. Typically, the measurements start by switching on both the light source and the receiver unit. Then the intensities of light are measured at different points across the detector plane. Several measurement procedures corresponding to different post processing techniques are plausible. For example an angular power density of the light scattered by particles contained in the fluid is measured either regardless, or as a function, of the wavelengths and or polarization angles of the illuminating beam. The scattering profiles of samples of fluids with and without bacteria substantially differ in specific angular aspects. Such differences are enhanced when the wavelengths of the illuminating light are for closely within the near infrared range. Similarly different polarization results in different scattering patterns as is induced by the bacteria.

**(iii) Post processing**

Scattering is defined by azimuth and angular elevation values. The measured scattering distribution function is basically a three dimensional mapping in which the intensity of the light impinging on a plane of the detector, are represented by the level of signal of the corresponding pixels. A scattering profile is obtained by either selecting a specified azimuth, or by averaging. The scattering profile along a given azimuth resembles the measured angular distribution of the scattered light along the corresponding direction across the detector plane. Averaged scattering profiles are obtained by averaging the measured angular distribution functions over a specified range of azimuth angles.

In general a post-processing step is based on a comparison between a calibration scale and the measured values and or values derived from the measurements. There are several different post processing techniques providing a calibration scale according to the present invention. An exemplary post processing technique embodying the present invention is hereinafter described. This technique is applicable with scattering measurements that are independent of the wavelength and the polarization of the illuminating light. The angular intensity profile of a first sample of urine filtered with a coarse filter and therefore is suspected as containing bacteria, is compared to the profile of a second sample of the same urine filtered with a fine filter. The second measured profile is calibrated or normalized such as by means of curve fitting, to the level of a pre-stored angular intensity profile of a sample of urine free of bacteria. Then the currently measured profile of the first sample is normalized by employing the same normalization factor. The normalized first profile is compared to a series of pre-stored scattering profiles of urine containing specific bacteria at specific bacterial concentrations. The urine is defined as being infected with bacteria when the differences between the measured profile of the sample matches a pre-stored trend of differences typically existing between profiles corresponding to infected and uninfected urine. The level of contamination is derived by taking the specific bacterial concentration of the pre-stored profile that best fits the measured profile in terms of curve fitting.

A set of reference scattering profiles for the detection of bacteria in a fluid such as urine, according to a preferred embodiment of the present invention is prepared as follows. A multiplicity of germ free urine samples is collected. Each of these samples is further divided into a few distinct sub-samples that are supplemented with specific bacteria each at a specific concentration. Angular scattering distribution is measured and is recorded for each urine sample. The recorded angular distribution functions are further grouped according to the level of bacterial contamination. A reference set of standard angular scattering distribution is formed by carrying out an ensemble averaging in correspondence with each calibrated bacterial concentration level. Calibrated scattering profiles are derived from these standard angular scattering distribution functions as described above. Bacterial concentration levels are measured in colony forming units (CFU) per milliliter (CFU/ml). A single bacterium is referred hereinafter as a CFU.

Detection of bacteria according to an embodiment of the present invention is accomplished by fitting a linear combination of a calibrated scattering profile of urine supplemented with bacteria and an uninfected calibrated scattering profile to the measured scattering profile. A linear combination of calibrated scattering profiles is given by the equation:

$S^c_i(x)=A_iS^f(x)+B_iS^b_i(x)$, where

x is the scattering angle;

$S^c_i(x)$ is the i'th outcome of the linear combination as a function of x;

$S^f(x)$ is the uninfected calibrated scattering profile as a function of x;

$S^b_i(x)$ is the i'th calibrated scattering profile of the set of calibrated scattering profiles infected with the bacteria or a specific mixture of bacteria as a function of x, the index i indicates specific bacterial concentration;

$A_i$ and $B_i$ are parameters the values of which are determined by curve fitting of the i'th linear combination to the measured profile. The curve fitting is accomplished by minimizing the sum of squared differences between the measured profile and the i'th linear combination of profiles at each value of x, by varying the values of the parameters $A_i$ and $B_i$. The j'th linear combination is chosen in which the best fit is achieved. The level of bacterial infection derived for this sample is the same as of the j'th calibrated scattering profile.

The measurements procedure and post processing techniques are further described in the examples below.

## Example 1

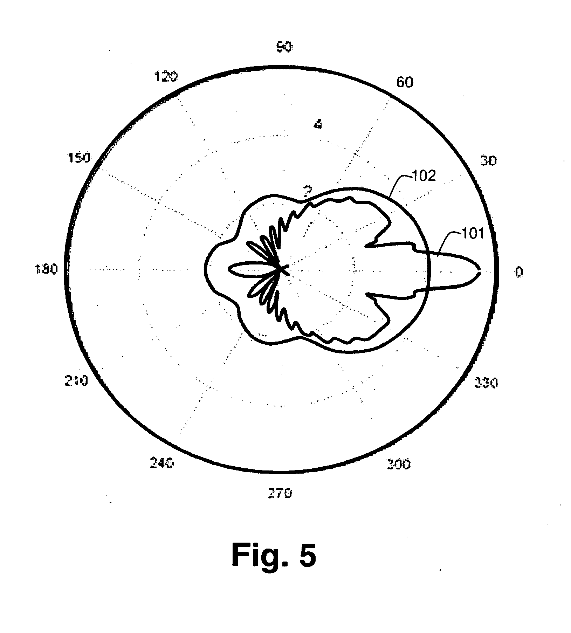

A simulated analysis of an angular scattering distribution was conducted: A synthetic model of urine was made in which 2-4 microns diameter spheres having the same dielectric constant as that of bacteria were included. Salt particles are represented by spheres having radius that is smaller than one micron and a matching dielectric constant. Calculations are based on the scattering Mie theory [H. C. van de Hulst. "Light scattering by small particles", John Wiley & Sons publishing, NY, 1957]. Reference is now made to **Fig. 5** showing a plot of the simulated angular scattering distribution function of suspensions of the two kinds of particles described above. The curve **101** represents the intensity of light scattered by the suspension of particles representing bacteria. Curve **102** corresponds to the intensity of light scattered by the small particle representing crystalline salt particles in urine. The intensity is shown in a logarithmic scale and is represented in polar coordinates versus the scattering angle. It is demonstrated that larger particles scatter mostly at small scattering angles, while scattering from smaller particles has a considerably broader angular distribution, which is of a slightly varying intensity at scattering angles within a considerable angular range centered at 0°.

## Example 2

Measurements described below were carried out by employing a system in which the light source is a laser diode of a specific wavelength and intensity and an CU having one cuvette. An elaborated model of urine containing bacteria was prepared and a scaled geometry of the system described in Fig. **1** to which reference is again made was employed as follows:

1) The dimensions of bacteria are defined in the literature.

2) The bacteria are randomly dispersed in the illuminated volume of the sample of fluid.

3) The light source of the model is a laser diode having the same features as the light source of the system employed in the actual measurements.

4) The angular intensities of scattered light for each single bacterium were calculated using the above mentioned Mie scattering model.

5) Scattered light rays are optically traced using a model of the optical unit employed for the actual measurements.

6) The power density of the scattered beam is calculated at the plane of the detector.

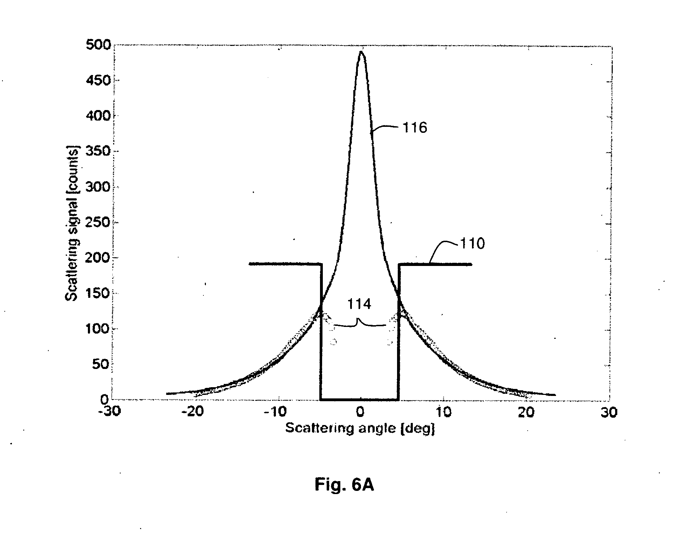

Reference is made to **Fig. 6A** showing plots of intensities of scattered light versus scattering angles of two typical samples of urine. Curve **110** represents synthetic transmittance of the cuvette considering the light reflector. Curve **114** represents measured scattering profile of a typical urine sample infected with E. coli at a bacterial concentration of $10^4$ CFU/ml. Curve **116** represents the simulated profile of same urine sample of which its total intensity is normalized to that of the measured profile.

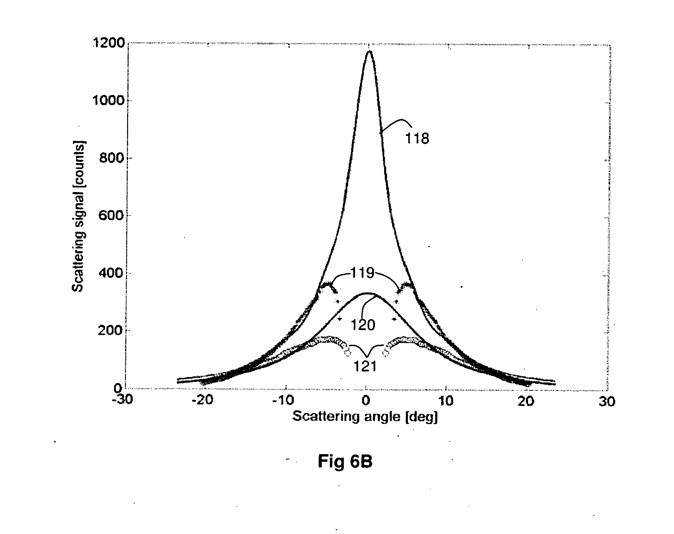

Reference is made to **Fig. 6B** in which a comparison between measured and simulated angular distributions of scattering intensity employing urine supplemented with bacteria are shown correspondingly. Measurements were carried out employing the same optical unit and CUs of a single cuvette configuration as is described in example 1 above. The simulated scattering intensities were calculated for bacteria statistically distributed within 2-4 microns in accordance with the elaborated model. The salt particles of the model are statistically distributed within 0 -1.5 micron range conforming to the measured data. Curves **118** and **119** represent simulated and measured signal intensities respectively as a function of the scattering angle employing urine samples with bacteria. Curves **120** and **121** show the simulated and measured scattering profiles of the urine samples without bacteria respectively.

**Example 3**

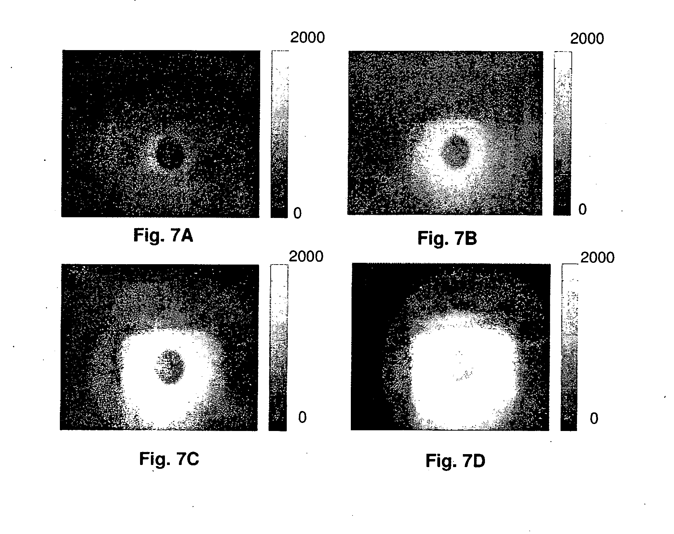

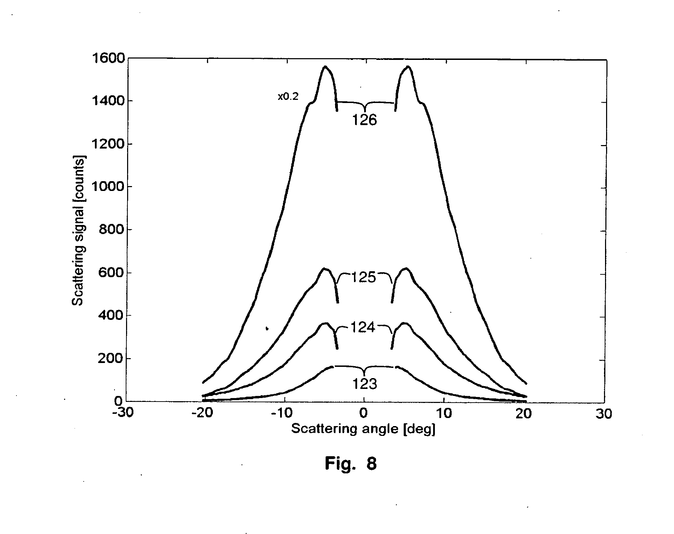

Light scattering measurements were conducted by employing the same optical unit and CUs as is described in example 2 above and several urine samples supplemented with bacteria at different bacterial concentration levels. Bacterial concentration levels were independently calibrated by employing incubation as in the prior art. Reference is now made to **Figs 7A - 7D** in which are shown typical angular scattering distribution functions of infected urine at bacterial concentration levels of $10^3$, $10^4$, $10^5$, and $10^6$ CFU/ml respectively. Scattering profiles were derived from these typical distribution functions by averaging over most of the azimuth range. In **Fig. 8** a graph comparing the scattering profiles corresponding to the measured angular distribution functions of **Figs 7A - 7D** are shown. Plots **123, 124, 125** and **126** represent these scattering profiles of infected urine at the same bacterial concentration levels as of **Figs 7A - 7D** respectively.

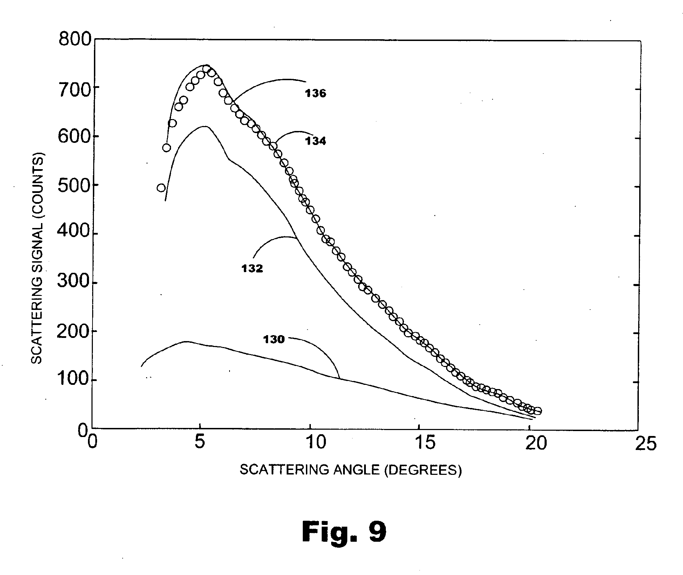

A typical urine sample known to be contaminated with e-coli was tested for a presence of bacteria by employing the same optical measuring device and CUs of single cuvette configuration as is described above. Scattering profile was measured and the detection of bacteria according to the preferred embodiment of the present invention as is herein described above has been conducted. Reference is now made to **Fig. 9** in which two calibrated scattering profiles of a set of calibrated profiles and the measured and fitted scattering profiles of this urine sample are correspondingly shown. Curve **130** represents the calibrated scattering profile of uninfected urine. Curve **132** represents one of the calibrated scattering profiles of infected urine (cleared of any salt particles) at a specific bacterial concentration level. Curve **134** represents the fitted scattering profile to the examined sample. Curve **136** represents the measured scattering profile of this sample under test. A considerably high level of matching is demonstrated over a significant range of scattering angles. The detected level of bacterial concentration in this examined sample deviated from the reference level by a few percent.

**Example 4**

An experiment carried out for estimating the sensitivity and specificity of the method of the invention is hereby described. A total of 364 samples of urine each received from a different patient in a clinical procedure as known, were examined for a presence of bacteria. A group of 158 samples were stored sealed at room temperature for a time not exceeding 6 hours from their reception time before being tested according to the method of the invention. A second group of 206 samples of urine were stored sealed at a temperature below 5°C for a time not exceeding 12 hours from the moment of their reception. Samples from the first group are referred hereinafter as fresh samples and samples from the second group are referred as refrigerated samples. The detection of bacteria was accomplished by fitting a linear combination of a calibrated scattering profile of urine supplemented with bacteria and an uninfected calibrated scattering profile to the measured scattering profile of each sample of both groups. The tests results are summarized in table a.

Table a. Detection of bacteria in urine according to the method of the present invention

|  | Refrigerated | Fresh |
|---|---|---|
| Clear (free of bacteria) | 95 | 138 |
| Contaminated with bacteria | 111 | 20 |
| Total | 206 | 158 |

The results received were compared to tests results carried by incubating the samples of urine as known. Table b summarizes the results of the experiment for both groups.

Table b. Sensitivity and specificity of the results shown in table a.

|  | Refrigerated | Fresh |
|---|---|---|
| Specificity | 83% | 90% |
| Sensitivity | 80% | 82% |

The sensitivity and specificity are defined by the following expressions,

$$\text{Sensitivity} = TP * 100 / (TP + FN),$$

and

$$\text{Specificity} = TN * 100 / (TN + FP),$$

where TP, FP, TN and FN means true positive, false positive, true negative and false negative respectively. The sensitivity and specificity of the detection in the refrigerated samples are somewhat lower than the same values received for the fresh samples. However the specificity values are significantly higher than the sensitivity received for both groups.

**Claims**

1. A method for detecting bacteria in a fluid comprising the steps of:

   a. filtering a sample of said fluid by means of a first filter thereby excluding particles larger than said bacteria;
   b. illuminating said sample of fluid using a collimated light beam;
   c. measuring the intensity of light scattered by said sample of fluid at least at one point, and
   d. comparing said scattered light to a calibration scale.

2. A method as in claim 1, further comprising

   i) filtering a second sample of said fluid by means of a second filter thereby excluding particles larger than a predefined limit smaller than said bacteria;
   ii) illuminating said second sample of fluid using a collimated light beam;
   iii) measuring the intensity of light scattered by said second sample of fluid at least at one point, and
   iv) comparing said scattered light to a calibration scale.

3. A method as in claims 1, further comprising associating a scattering profile with said measured intensity, wherein said measuring accomplished at least at two different points.

4. A method as in claim 3, further comprising matching to said associated scattering profile any item selected from a list of items consisting of pre-stored calibrated scattering profiles and linear combinations of pre-stored calibrated scattering profiles.

5. A method as in claim 1, further comprising polarising said collimated light beam.

6. A method as in claim 1, wherein said measuring is carried out regarding at least at one specific wavelength.

7. A method as in claim 1, wherein said illuminating is effected by at least one laser diode.

**Patentansprüche**

1. Eine Methode zum Nachweis von Bakterien in einer Flüssigkeit, die folgende Schritte umfasst:

   a) Filtrieren einer Probe der besagten Flüssigkeit mittels eines ersten Filters, wodurch Teilchen, die größer als die besagten Bakterien sind, entfernt werden;
   b) Beleuchten der besagten Flüssigkeitsprobe unter Verwendung eines parallelen Lichtstrahls;
   c) Messen der Intensität des Lichts, das von der besagten Flüssigkeitsprobe gestreut wird, an mindestens einem Punkt, und
   d) Vergleich des besagten Streulichts mit einer Kalibrierungsskala.

2. Eine Methode gemäß Anspruch 1, die überdies folgende Schritte umfasst:

   i) Filtrieren einer zweiten Probe der besagten Flüssigkeit mittels eines zweiten Filters, wodurch Teilchen, die größer als ein vordefinierter Grenzwert sind, der kleiner als die besagten Bakterien ist, entfernt werden;
   ii) Beleuchten der besagten zweiten Flüssigkeitsprobe unter Verwendung eines parallelen Lichtstrahls;

iii) Messen der Intensität des Lichts, das von der besagten zweiten Flüssigkeitsprobe gestreut wird, an mindestens einem Punkt, und
iv) Vergleich des besagten Streulichts mit einer Kalibrierungsskala.

**3.** Eine Methode gemäß Anspruch 1, die überdies die Verbindung eines Streuungsprofils mit der besagten gemessenen Intensität umfasst, wobei die besagte Messung an mindestens zwei verschiedenen Punkten durchgeführt wird.

**4.** Eine Methode gemäß Anspruch 3, die überdies die Zuordnung irgendeiner Größe, die aus einer Liste von Größen ausgewählt wurde, zu dem besagten zugehörigen Streuungsprofil beinhaltet, wobei diese Liste aus vorgespeicherten kalibrierten Streuungsprofilen und linearen Kombinationen von vorgespeicherten kalibrierten Streuungsprofilen besteht.

**5.** Eine Methode gemäß Anspruch 1, die überdies die Polarisation des besagten parallelen Lichtstrahls umfasst.

**6.** Eine Methode gemäß Anspruch 1, worin die besagte Messung im Hinblick auf mindestens eine spezifische Wellenlänge durchgeführt wird.

**7.** Eine Methode gemäß Anspruch 1, worin die besagte Beleuchtung mit mindestens einer Laserdiode erfolgt.

**Revendications**

**1.** Procédé de détection de bactéries dans un liquide comprenant les étapes de :

a. filtrage d'un échantillon dudit liquide au moyen d'un premier filtre, excluant ainsi les particules plus grandes que lesdites bactéries ;
b. illumination dudit échantillon de liquide en utilisant un faisceau lumineux collimaté ;
c. mesure de l'intensité lumineuse diffusée par ledit échantillon de liquide à au moins un point ; et
d. comparaison de ladite lumière diffusée par rapport à une échelle d'étalonnage.

**2.** Procédé selon la revendication 1, comprenant en outre

i) le filtrage d'un deuxième échantillon dudit liquide au moyen d'un deuxième filtre, excluant ainsi les particules plus grandes qu'une limite prédéfinie plus petite pour lesdites bactéries ;
ii) illumination dudit deuxième échantillon de liquide en utilisant un faisceau lumineux collimaté ;
iii) mesure de l'intensité lumineuse diffusée par ledit deuxième échantillon de liquide à au moins un point, et
iv) comparaison de ladite lumière diffusée par rapport à une échelle d'étalonnage.

**3.** Procédé selon les revendications 1, consistant en outre en l'association d'un profil de diffusion avec ladite intensité mesurée, selon lequel ladite mesure est effectuée à au moins deux points différents.

**4.** Procédé selon la revendication 3, comprenant en outre l'appariement audit profil de diffusion associé tout élément sélectionné à partir d'une liste d'éléments de profils de diffusion étalonnés préstockés et de combinaisons linéaires de profils de difussion étalonnés préstockés.

**5.** Procédé selon la revendication 1, comprenant en outre la polarisation dudit faisceau lumineux collimaté.

**6.** Procédé selon la revendication 1, dans lequel ladite mesure est effectuée relativement à au moins une longueur d'onde spécifique.

**7.** Procédé selon la revendication 1, dans lequel ladite illumination est effectuée par au moins une diode laser.

Fig. 1

Fig. 2

Fig. 3

Fig. 4A

Fig. 4B

Fig. 4C

Fig. 4D

**Fig. 5**

**Fig. 6A**

**Fig 6B**

Fig. 7A

Fig. 7B

Fig. 7C

Fig. 7D

**Fig. 8**

**Fig. 9**

**REFERENCES CITED IN THE DESCRIPTION**

**Patent documents cited in the description**

- EP 0320154 A1

- EP 1136563 A

**Non-patent literature cited in the description**

- **H. C. van de Hulst.** Light scattering by small particles. John Wiley & Sons, 1957